# EUROPÄISCHE PATENTSCHRIFT

(11) **EP 0 857 062 B1**
(45) Veröffentlichungstag und Bekanntmachung des Hinweises auf die Patenterteilung: **17.04.2002**
(21) Anmeldenummer: 96937214.3
(22) Anmeldetag: 23.10.1996
(51) Int. Cl.: A61K 9/20

(54) **VERFAHREN ZUR HERSTELLUNG VON MEHRSCHICHTIGEN, FESTEN ARZNEIFORMEN ZUR ORALEN ODER REKTALEN VERABREICHUNG**
METHOD OF PRODUCING MULTI-LAYER MEDICAMENTS IN SOLID FORM FOR ORAL OR RECTAL ADMINISTRATION
PROCEDE DE PREPARATION DE FORMES GALENIQUES SOLIDES MULTICOUCHES POUR ADMINISTRATION ORALE OU RECTALE

(30) Priorität: 23.10.1995 DE 19539361
(43) Veröffentlichungstag der Anmeldung: 12.08.1998
(73) Patentinhaber: BASF AKTIENGESELLSCHAFT, 67056 Ludwigshafen (DE)
(72) Erfinder: BREITENBACH, Jörg, D-68199 Mannheim (DE); HÄRTL, Axel, Paul, D-67246 Dirmstein (DE); HOFMANN, Jürgen, D-67069 Ludwigshafen (DE); ROSENBERG, Joerg, D-67158 Ellerstadt (DE); SCHIESSL, Michael, D-67454 Hassloch (DE); ZETTLER, Hans, Dieter, D-67269 Grünstadt (DE)
(74) Vertreter: Kinzebach, Werner, Dr.
(86) Internationale Anmeldenummer: EP9604601
(87) Internationale Veröffentlichungsnummer: WO9715293

(56) Entgegenhaltungen:
- WO-A-91/10425

## Beschreibung

Die vorliegende Erfindung betrifft ein Verfahren zur Herstellung von mehrschichtigen, festen Arzneiformen zur oralen oder rektalen Verabreichung sowie die mit dem erfindungsgemäßen Verfahren erhältlich Arzneiformen.

Arzneiformen aus mehreren Schichten, beispielsweise Manteltabletten, Mehrschichttabletten, Manteldragees und Mehrschichtdragees, finden immer häufigere Anwendung, beispielsweise um miteinander unverträgliche Wirkstoffe zu kombinieren oder um die Abgabe einer Initial- und Erhaltungsdosis bei Retardarzneiformen zu bewerkstelligen. Die Herstellung derartiger Arzneiformen erfolgt nach klassischen Methoden. So erfolgt die Herstellung von Manteltabletten durch Trockendragierung und die Herstellung von Mehrschichttabletten durch Verpressen von zwei oder mehreren Schichten von Granulaten. Hierfür sind Spezialmaschinen erforderlich, die in ihrer Arbeitsweise den üblichen Rundläufern ähneln, wobei mindestens zwei Füll- und Preßstationen Voraussetzung sind. Diese herkömmlichen Verfahren sind deshalb aufwendig und kostenintensiv.

Seit einiger Zeit ist ein Verfahren zur Herstellung von Tabletten bekannt, das im Vergleich zu dem vielstufigen, diskontinuierlichen klassischen Tablettierungsverfahren wesentlich einfacher ist. Es besteht darin, daß man den Wirkstoff in einem polymeren Bindemittel aufnimmt, die wirkstoffhaltige Polymerschmelze extrudiert und den aus dem Extruder austretenden Strang in geeigneter Weise formt, siehe beispielsweise EP-A-240 904 und 240 906.

Aus der Kunststofftechnik ist die sogenannte Coextrusion bekannt, bei der Schmelzeströme aus mehreren Extrudern in einem Werkzeug so zusammengeführt werden, daß sich der gewünschte Schichtaufbau aus verschiedenen Thermoplasten ergibt. In der Pharmaindustrie ist der Einsatz der Coextrusion hauptsächlich auf die Herstellung von Verpackungsfolien begrenzt. Darüber hinaus ist die Herstellung von Polymerkapseln und gecoateten Wirkstoffen in Form eines Fischmedikamentes und eines Implantats bekannt:

Die WO-A-89/12442 beschreibt eine pharmazeutische Dosierungsform für die Medikation von Fischen. Arzneimittel werden Fischen in der Regel über das Futter verabreicht, d.h. das Arzneimittel wird mit dem Futter vermischt. Dabei bestand das Problem, daß das arzneimittelhaltige Futter aufgrund seines Geschmackes von den Fischen nicht angenommen wurde. Das hatte zur Folge, daß ein großer Teil des arzneimittelhaltigen Futters über längere Zeit im Wasser verblieb, ungenutzt blieb und absinken konnte. Dies führte zu einer unerwünschten Freisetzung des Arzneimittels in das Wasser, was natürlich eine Verschmutzung des Wassers zur Folge hatte.

Zur Lösung dieses Problems schlägt die WO 89/12442 eine durch Coextrusion erhaltene Dosierungsform vor, die aus einer äußeren Schicht besteht, welche eine innere Kammer umgibt. Die äußere Schicht besteht aus einem Stärkederivat, das ein geeignetes tierisches oder pflanzliches Material enthält, um die Dosierungsform den Fischen annehmbar zu machen. Außerdem ist die äußere Schicht für Wasser und den in der inneren Kammer enthaltenen Wirkstoff impermeabel. Die innere Kammer enthält den Wirkstoff in einer viskosen Suspension, welche die Kammer nur teilweise ausfüllt. Dadurch wird ein Luftraum bereitgestellt, welcher der Dosierungsform den nötigen Auftrieb verleiht, damit sie nicht absinkt, sondern im Wasser schwimmt.

Die US-A-5,283,187 beschreibt ein Implantat, das als Wirkstoff eine Zellsuspension enthält, welche in eine semipermeable Polymermembran eingeschlossen ist. Die Herstellung des Implantats erfolgt durch Coextrusion der Zellsuspension mit einer Lösung des Polymers in einem geeigneten mit Wasser mischbaren organischen Lösungsmittel. Das Polymer muß so gewählt sein, daß es beim Extrudieren koaguliert und ein Netzwerk aus Kanälen bildet, so daß die Membran semipermeabel wird.

Die EP-A-303 306 beschreibt ein zylindrisches Implantat, das einen Kern aus einem Ethylen/Vinylacetat-Copolymer mit einem Schmelzindex von mehr als 10 g/10 min und einem Vinylacetatgehalt von mindestens 20 Gew.-% aufweist. Der Kern ist von einer Membran mit einer Dicke von 50 bis 250 µM umgeben, welche ebenfalls aus einem Ethylen/Vinylacetat-Copolymer besteht. Dieses Polymer besitzt allerdings einen Schmelzindex von weniger als 10 g/10 min und einen Vinylacetatgehalt von weniger als 20 Gew.-%. Die Membran dient dazu, die Freisetzung des im Kern enthaltenen Wirkstoffes, ein Kontrazeptivum, so zu regulieren, daß dieses in einer Tagesdosis von 15 bis 30 µg über einen Zeitraum von wenigstens 2 Jahren freigesetzt wird. Das Implantat wird durch Coextrusion der beiden Polymerschichten hergestellt.

Die oben erwähnten Implantate werden parenteral, beispielsweise subkutan, verabreicht. Die äußere Schicht der Implantate ist so beschaffen, daß sie sich in den Körperflüssigkeiten nicht auflöst und das Implantat daher auf einfache Weise wieder aus dem Körper entfernt werden kann.

Demgegenüber bestehen ganz andere Anforderungen an eine oral oder rektal verabreichbare Arzneiform, welche die gezielte Einstellung der gewünschten Freisetzungscharakteristik des Wirkstoffes erlauben soll. Eine derartige Arzneiform soll, im Vergleich zu einem Implantat, den Wirkstoff relativ rasch in der gewünschten Weise und am gewünschten Ort freisetzen und sich zweckmäßigerweise in Körperflüssigkeit auflösen.

Der vorliegenden Erfindung liegt daher die Aufgabe zugrunde, feste, oral oder rektal verabreichbare Arzneiformen sowie ein Verfahren zu ihrer Herstellung zur Verfügung zu stellen, das es erlaubt, die Arzneiform auf einfache und schonende Weise herzustellen und für die gewünschte Freisetzungscharakteristik zu sorgen.

Überraschenderweise wurde nun gefunden, daß diese Aufgabe durch eine mehrschichtige feste Arzneiform gelöst wird, die durch Coextrusion von zwei Massen aus einem pharmazeutisch akzeptablen thermoplastischen Polymer, von denen wenigstens eine einen pharmazeutischen Wirkstoff enthält, erhältlich ist.

Gegenstand der vorliegenden Erfindung ist daher ein Verfahren zur Herstellung mehrschichtiger fester Arzneiformen zur oralen oder rektalen Verabreichung, das dadurch gekennzeichnet ist, daß man mindestens zwei Massen, die jeweils ein thermoplastisches, physiologisch akzeptables und in physiologischer Umgebung lösliches oder quellbares polymeres Bindemittel umfassen und von denen wenigstens eine einen pharmazeutischen Wirkstoff enthält, coextrudiert und das coextrudierte mehrschichtige Material zu der gewünschten Arzneiform formt, sowie die Arzneiformen, die nach diesem Verfahren erhältlich sind.

Es zeigt:
- Fig. 1: eine schematische Schnittdarstellung der Coextrusion und Formung von Tabletten mit einer Formwalze
- Fig. 2: eine schematische Schnittdarstellung der Formung von Tabletten mit einer Quetschvorrichtung.

Zu festen Arzneiformen für die orale und rektale Verabreichung zählen insbesondere Tabletten, Dragees, Pastillen und Pellets sowie Suppositorien.

Die erfindungsgemäß hergestellten Arzneiformen sind vorzugsweise so beschaffen, daß die äußere Schicht (die äußeren Schichten) keine Membran darstellt (darstellen), sondern in der Körperflüssigkeit löslich und/oder quellbar ist (sind) und gegebenenfalls eine Schutz- oder Haftschicht darstellt.

Die erfindungsgemäß herstellbaren Arzneiformen umfassen vorzugsweise zwei oder drei Schichten. Sie können in offener oder geschlossener Form vorliegen, insbesondere als offene oder geschlossene Mehrschichttablette.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, daß zwei miteinander unverträgliche Wirkstoffe verarbeitet werden können oder daß die Freisetzungscharakteristik des Wirkstoffes gesteuert werden kann. Beispielsweise ist es möglich, eine Initialdosis zur Aufnahme eines Wirkstoffes in eine der äußeren Schichten und eine Erhaltungsdosis durch Aufnahme des Wirkstoffes in die innere(n) Schicht(en) bereitzustellen.

Die Dicke der Schichten kann in Abhängigkeit von der gewünschten Freisetzungscharakteristik gewählt werden. Je dicker die Schicht ist, umso länger wird die Freisetzung des Wirkstoffes verzögert, d.h. umso länger hält die Wirkung an.

Die erfindungsgemäßen Arzneiformen sind insbesondere geeignet, um das sogenannte Colon-Targeting zu bewerkstelligen. Zu diesem Zweck kann die Freisetzung des Wirkstoffes durch die Wahl entsprechender Materialien zeitlich, pH-abhängig oder enzymabhängig gesteuert werden. Die zeitliche Steuerung kann beispielsweise durch die Dicke einer Schicht und/oder rasch oder langsam sich auflösende Materialien erfolgen. Eine relativ rasche Auflösung erfolgt beispielsweise mit Polyvinylpyrrolidon, eine relativ langsame Auflösung mit Ethylcellulose, Polyacrylaten oder Polymethacrylaten (Eudragit RL, RS).

Die pH-abhängige Steuerung kann durch Verwendung magensaftlöslicher (z.B. Polyvinylpyrrolidon) und/oder magensaftresistenter, darmsaftlöslicher Materialien (z.B. Cellulosephthalate, Polyacrylate oder Methacrylate (Endragit L 30 D oder S)) erfolgen.

Die enzymabhängige Steuerung kann beispielsweise durch Verwendung von Materialien erfolgen, die den Wirkstoff erst unter Einwirkung von Enzymen im Darm freisetzen, wie Galactomannane.

Die Herstellung der Arzneiformen erfolgt ausgehend von mindestens zwei separaten Massen (Mischungen), welche jeweils mindestens ein thermoplastisches, pharmakologisch akzeptables polymeres Bindemittel, ggf. einen oder mehrere pharmazeutische Wirkstoffe sowie einen oder mehrere übliche Hilfsstoffe umfassen und welche durch Schmelzen oder Erweichen mindestens einer Komponente teigig bis zähflüssig (thermoplastisch) und daher extrudierbar werden. Die Glasübergangstemperatur der Masse liegt unter der Zersetzungstemperatur aller in der Masse enthaltenen Komponenten. Das Bindemittel soll vorzugsweise in physiologischer Umgebung löslich oder quellbar sein. Beispiele für geeignete Bindemittel sind Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, Stärke, Stärkederivate, z.B. Maltodextrine, Zuckeralkohole, wie Mannit oder Palatinose, und Mannane, insbesondere Galactomannane. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Bevorzugte Bindemittel zur Aufnahme eines Wirkstoffes sind Polyvinylpyrrolidon, Copolymerisate von N-Vinylpyrrolidon und Vinylestern und Hydroxyalkylacrylate.

Bevorzugte Bindemittel für wirkstofffreie Schichten sind in wäßrigem Medium oder bei pH <5 unlösliche Bindemittel, insbesondere Hydroxyalkylcellulosen, Alkylcellulosen, Hydroxyalkyl-Alkylcellulosen, Polyacrylate, Cellulosephthalate, Polylactide und Galactomannane.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß daher unter 180°C, vorzugsweise unter 130°C liegen. Erforderlichenfalls wird sie durch übliche pharmakologisch akzeptable weichmachende Hilfsstoffe herabgesetzt, wie langkettige Alkohole, Ethylenglykol, Propylenglykol, Glycerin, Trimethylolpropan, Triethylenglykol, Zuckeralohole, z.B. Butandiole, Pentanole, wie Pentaerythrit oder Hexanole, Polyethylenglykole, Polypropylenglykole, Polyethylen-propylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) Fettsäureester, wie Glycerinmono-, Glycerindi- oder Glycerintriacetat oder Natriumdiethylsulfosuccinat. Die Konzentration an Weichmacher beträgt im allgemeinen 0,5 bis 15, vorzugsweise 0,5 bis 5 Gew.-%, bezogen auf das Gesamtgewicht der Masse für die jeweilige Schicht. Vorzugsweise umfaßt die Mischung keinen Weichmacher.

Übliche galenische Hilfsstoffe, deren Gesamtmenge bis zu 100 Gew.-% bezogen auf das Polymerisat, betragen kann, sind z.B.

Streckmittel bzw. Füllstoffe, wie Silikate oder Kieselerde, Magnesiumoxid, Aluminiumoxid, Titanoxid, Stearinsäure oder deren Salze, z.B. das Magnesium- oder Kalziumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht der Masse für die jeweilige Schicht;

Schmiermittel, wie Aluminium- und Calciumstearat, Talcum und Silicone, in einer Konzentration von 0,1 bis 5, vorzugsweise 0,1 bis 3 Gew.-%, bezogen auf das Gesamtgewicht der Masse für die jeweilige Schicht;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht der Masse für die jeweilige Schicht, bevorzugt sind;

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z.B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetracaetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J. L. Ford, Pharm. Acta Helv. 61, 69-88 (1986) angegeben.

Einzige Voraussetzung für die Eignung von Hilfsstoffen ist eine ausreichende Temperaturstabilität.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen, z.B. Ibuprofen/-Coffein, können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z.B. Adenin, Cholin, Pantothensäure, Biotin, Adenylsäure, Folsäure, Orotsäure, Pangamsäure, Carnitin, p-Aminobenzoesäure, myo-Inosit und Liponsäure, sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika.

Das erfindungsgemäße Verfahren ist beispielsweise zur Verarbeitung folgender Wirkstoffe geeignet:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxocyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Ginkgo Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamide, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpiridid, Tamoxifen, Tegafur, Teprenon, Terazosin, Terbutalin, Terfenadin, Tetracyclin, Theophyllin, Thiamin, Tiolopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Im einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Vor der Koextrusion muß die Masse für jede Schicht der Arzneiform separat zubereitet werden. Zu diesem Zweck werden die Ausgangskomponenten lösungsmittelfrei in einem separaten Extruder oder Schmelzebehälter mit nachgeschalteter Zahnradpumpe verarbeitet. Dabei können die Komponenten einzeln oder als Trockenvormischung kontinuierlich (z.B. über Differentialdosierungswaagen) eingespeist werden. In dem Extruder oder Schmelzebehälter erfolgt dann ein Vermischen und/oder Erweichen oder Aufschmelzen der Masse. Wenn man wünscht, einen insbesondere temperaturempfindlichen Wirkstoff einzuarbeiten, so wird dieser zweckmäßigerweise erst nach dem Erweichen oder Aufschmelzen der Masse zugegeben und durch Längs- und Quervermischen im Extruder oder in einem Kneter oder Mischreaktor eingearbeitet und mit der Masse homogenisiert. Für die Zubereitung der Masse ist ein Extruder, insbesondere ein Zweischneckenextruder oder Einschneckenextruder mit Mischabteil, besonders zweckmäßig, da hierbei unter materialspezifisch optimalen Bedingungen gearbeitet werden kann. Beispielsweise kann für jede Schicht eine unterschiedliche Verarbeitungstemperatur gewählt werden.

Die geschmolzenen oder plastischen Massen aus den einzelnen Extrudern oder anderen Aggregaten werden in ein gemeinsames Koextrusionswerkzeug geführt, ausgeformt und ausgetragen. Die Form der Koextrusionswerkzeuge richtet sich nach der gewünschten Arzneiform. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten Arzneiform.

Nach dem Austrag aus dem Koextrusionswerkzeug erfolgt eine Formung zu der gewünschten Arzneiform. Dabei kann eine Vielzahl von Formen je nach Koextrusionswerkzeug und Art der Formung erzeugt werden. Beispielsweise kann man aus einem Strang, der aus einem Breitschlitzwerkzeug austritt und der insbesondere zwei oder drei Schichten aufweist, durch Ausstanzen oder Ausschneiden z.B. mittels Glühdraht, offene Mehrschichttabletten herstellen. Alternativ können offene Mehrschichttabletten über ein Werkzeug mit kreisringspaltförmigem Austrittsquerschnitt über einen Heißabschlag, d.h. durch Zerschneiden bzw. Zerhacken des Stranges unmittelbar nach dem Austritt aus der Düse, oder vorzugsweise über einen Kaltabschlag, d.h. durch Zerschneiden bzw. Zerhacken des Stranges nach zumindest teilweisem Abkühlen, separiert werden.

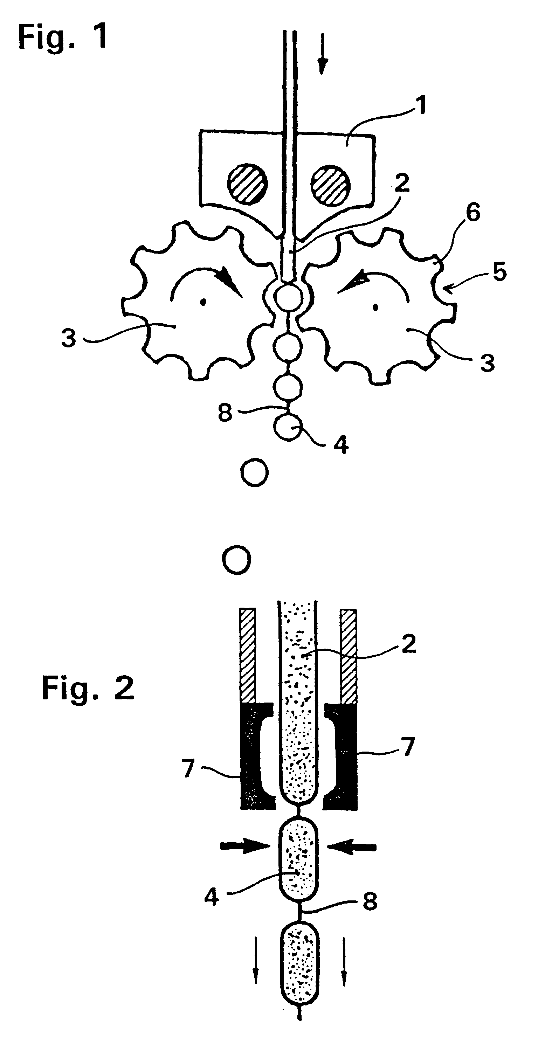

Geschlossene Arzneiformen, d.h. Arzneiformen, bei denen die wirkstoffhaltige Schicht vollständig von einer wirkstofffreien Schicht umgeben ist, erhält man insbesondere über ein Werkzeug mit kreisringspaltförmigem Austrittsquerschnitt durch Behandlung des Stranges in einer geeigneten Quetschvorrichtung, wie sie beispielsweise in den Figuren 1 und 2, die in den nachfolgenden Beispielen erläutert wird, gezeigt ist. Dabei ist es von Vorteil, wenn bei bereits abgekühlter Außenschicht die Innenschicht der Mehrschichttablette beim Eintritt in die Quetschvorrichtung noch plastisch verformbar ist. Auf diese Weise lassen sich insbesondere Tabletten, vorzugsweise Oblong-Tabletten, Dragees, Pastillen und Pellets herstellen.

Die mehrschichtigen Arzneiformen können in einem nachgeschalteten Verfahrensschritt nach üblichen Methoden rundiert und/oder mit einem Coating versehen werden. Das Rundieren erfolgt vorzugsweise durch Walzen, Bänder und Pressen und das Coating durch Behandlung in Dragierkesseln, Wirbelschicht-, Fließbett- oder Flugschichtapparatur.

Mit dem erfindungsgemäßen Verfahren ist es somit möglich, auf besonders einfache und schonende Weise feste Arzneiformen zur oralen und rektalen Verabreichung herzustellen. Außerdem bietet das Verfahren die Möglichkeit, die gewünschte Freisetzungscharakteristik durch die Wahl der Arzneiform und des Aufbaus davon und durch die Wahl des polymeren Bindemittels in einem weiten Bereich einzustellen.

Die nachfolgenden Beispiele erläutern die Erfindung, ohne sie zu beschränken.

### Beispiel 1

Über einen zweiwelligen Extruder (Typ ZSK 25) werden 10 kg/h Hydroxypropylcellulose (Klucel F) kontinuierlich dosiert und aufgeschmolzen. Parallel dazu werden über einen weiteren 2-welligen Extruder (ZSK 30) 30 kg/h Polyvinylpyrrolidon (PVP), das als Wirkstoff 30 Gew.-% Ibuprofen enthält, aufbereitet. Diese Stränge werden so gerührt, daß über eine konzentrische kreisringspaltförmige Koextrusionsdüse ein Strang, bestehend aus einem wirkstoffhaltigen PVP-Kern und einer Klucel-Hülle, unter folgenden Bedingungen extrudiert wird:

| | |
|---|---|
| Extruder ZSK 30: | Extruder ZSK 25: |
| Schuß 1: 43°C | Schuß 1: 70°C |
| Schuß 2: 57°C | Schuß 2: 120°C |
| Schuß 3: 120°C | Schuß 3: 110°C |
| Schuß 4: 100°C | Schuß 4: 100°C |
| Schuß 5: 100°C | Schuß 5: 100°C |
| Kopf : 100°C | Kopf : 110°C |
| Düse : 100°C | Düse : 100°C |

Dieser Strang wird dann über die in den Figuren 1 und 2 gezeigte Abquetschvorrichtung in geschlossene Oblong-Tabletten separiert. In Figur 1 ist die Koextrusionsdüse mit 1 bezeichnet. Der aus der Düse austretende Strang 2 (die einzelnen Schichten sind in der Figur nicht gezeigt) wird in einen Kalander mit zwei gegenläufig rotierenden Formwalzen 3 geführt. Die Formwalzen weisen Vertiefungen 5 auf, welche durch Stege 6 getrennt sind. Der Abstand der Formwalzen 3 ist so gewählt, daß sie einander an einer Linie auf einem der Stege 6 berühren oder daß lediglich ein sehr geringer Abstand besteht. Die Form der Vertiefungen 5 ist in einem weiten Bereich wählbar, so daß auf diese Weise zahlreiche Arzneiformen hergestellt werden können.

Der aus der Koextrusionsdüse 1 austretende Strang 2 wird in den Vertiefungen 5 aufgenommen und durch die Stege 6 zu einzelnen Arzneiformen separiert. Mit der in Figur 1 gezeigten Vorrichtung erhält man auf diese Weise Oblong-Tabletten 4, die miteinander noch über den Grat 8 verbunden sind.

Alternativ kann das Quetschen mit der in Figur 2 gezeigten Vorrichtung erfolgen. Der aus der Koextrusionsdüse austretende Produktstrang 2 wird in eine Vorrichtung geführt, welche zwei einander gegenüberliegende und den Strang 2 umschließende Quetschbalken 7 aufweist. Die Quetschbalken 7 sind senkrecht zum Strang 2 beweglich (in Fig. 2 durch die Pfeile angedeutet) und weisen einander gegenüberliegende Vertiefungen, entsprechend den Vertiefungen auf den Kallanderwalzen 3 in der Figur 1, auf. Um die Arzneiform zu separieren, werden die Quetschbalken 7 in Richtung auf den Strang 2 bewegt, bis sie einander berühren oder einen nur noch sehr geringen Abstand besitzen. Auf diese Weise kommt es zu einer Separierung der Arzneiform, wobei die einzelnen Arzneiformen noch über einen Grat 8 miteinander verbunden sind. Mit der in Figur 2 gezeigten Vorrichtung erhält man ebenfalls geschlossene Oblong-Tabletten.

Die erhaltenen Oblong-Tabletten können in üblicher Weise, beispielsweise in rotierenden Kesseln, entgratet werden.

Die Klucel-Außenhülle der erhaltenen Oblong-Tabletten bewirkt eine langsamere Freisetzung des im PVP-Kern dispers verteilten Wirkstoffes.

### Beispiel 2

Nach den in Beispiel 1 angegebenen Verfahren und mit den dort beschriebenen Materialien, wobei die Hydroxypropylcellulose 5 % Coffein enthält, erhält man Tabletten, die im Kern Ibuprofen und in der Außenschicht Coffein enthalten.

### Beispiel 3

Über einen zweiwelligen Extruder (Typ ZSK 25) werden 10 kg/h eines Gemisches aus Hydroxypropylcellulose und Ethylcellulose im Gewichtsverhältnis 8:1 kontinuierlich dosiert und aufgeschmolzen. Parallel dazu werden über einen weiteren zweiwelligen Extruder (ZSK 30) 15 kg/h Polyvinylpyrrolidon, das als Wirkstoff 40 Gew.-% Paracetamol enthält, aufbereitet. In einem dritten Strang werden über eine Zahnradpumpe 15 kg/h Hydroxypropylcelluloseschmelze, die als Wirkstoff 40 Gew.-% Paracetamol enthält, fördert.

Diese Stränge werden so in eine konzentrische kreisringspaltförmige Koextrusionsdüse geführt, daß ein Strang extrudiert wird, der aus einem Hydroxypropylcellulosekern mit geringer Freisetzungsrate, einer umgebenden Schicht von Polyvinylpyrrolidon hoher Freisetzungsrate und einer Hydroxypropylcellulose/Ethylcellulose-Hülle besteht (die Extrusionsbedingungen sind wie in Beispiel 1 angegeben).

Der extrudierte Strang wird über die in Figur 1 oder Figur 2 gezeigte Abquetschvorrichtung in einzelne geschlossene Tabletten separiert.

Durch die erhaltene Mehrschichttablette kann die Freisetzungskinetik des Wirkstoffes zur Erhöhung der Patientencompliance optimal gesteuert werden.

### Beispiel 4

In einem zweiwelligen Extruder (Typ ZSK 30) werden 15 kg/h Polyvinylpyrrolidon, das als Wirkstoff 30 Gew.-% Nifedipin enthält, aufbereitet. In einem weiteren Strang werden parallel dazu über eine Zahnradpumpe 15 kg/h Hydroxypropylcelluloseschmelze, die als Wirkstoff 40 Gew.-% Nifedipin enthält, gefördert.

Die beiden Stränge werden so über eine Breitschlitzdüse (3 Schlitze) geführt, daß eine Masse mit Sandwichstruktur extrudiert wird, die aus einer Hydroxypropylcelluloseschicht mit geringer Freisetzungsrate, die auf beiden Seiten von einer Polyvinylpyrrolidonschicht mit hoher Freisetzungsrate umgeben ist, besteht (Extrusionsbedingungen wie in Beispiel 1 angegeben).

Der extrudierte Strang wird über eine Ausstanzvorrichtung in offene Mehrschichttabletten separiert.

In einem anschließenden Verfahrensschritt können die erhaltenen offenen Mehrschichttabletten in einem Dragierkessel mit einem Acrylsäurecopolymer umhüllt werden.

Durch die Sandwichstruktur der Mehrschichttablette kann die Freisetzungskinetik des Wirkstoffes zur Erhöhung der Patientencompliance optimal gesteuert werden.

### Beispiel 5

Man erzeugt nach dem in Beispiel 1 angegebenen Verfahren und mit den dort angegebenen Materialien einen Strang, der über eine geeignete Kaltabschlagvorrichtung in offene Mehrschichttabletten separiert wird. Die Klucel-Außenhülle bewirkt eine langsamere Freisetzung des im PVP-Kern dispers verteilten Wirkstoffes.

### Beispiel 6

Mit den in Beispiel 3 beschriebenen Materialien und nach den dort beschriebenen Verfahren erzeugt man einen Strang aus einem Hydroxypropylcellulose-Kern geringer Freisetzungsrate, einer umgebenden Schicht von Polyvinylpyrrolidon mit hoher Freisetzungsrate und einer äußeren Schicht aus Hydroxypropylcellulose/Ethylcellulose. Dieser Strang wird über eine Kaltabschlagvorrichtung in einzelne offene Mehrschichttabletten separiert.

Durch diese Anordnung der Mehrschichttablette kann die Freisetzungskinetik zur Erhöhung der Patientencompliance optimal gesteuert werden.

### Beispiel 7

Über einen zweiwelligen Extruder (Typ ZSK 25) werden 10 kg/h Polylactid kontinuierlich dosiert und aufgeschmolzen. Parallel dazu werden in einem weiteren zweiwelligen Extruder (Typ ZSK 30) 30 kg/h Polyvinylpyrrolidon, das als Wirkstoff 40 Gew.-% Ibuprofen enthält, aufbereitet. Die beiden Stränge werden über eine kreisringspaltförmige Koextrusionsdüse so geführt, daß man einen Strang erhält, der aus einem wirkstoffhaltigen PVP-Kern und einer Polylactid-Hülle besteht (Estrusionsbedingungen wie in Beispiel 1 angegeben).

Dieser Strang wird über eine Kaltabschlagvorrichtung in einzelne offene Mehrschichttabletten separiert.

Die Polylactidhülle ist hydrolysestabil und kann sowohl enzymatisch als auch hydrolytisch zersetzt werden, so daß der Wirkstoff aus der Kernmatrix freigesetzt werden kann.

### Beispiel 8

Über einen zweiwelligen Extruder (ZSK 25) werden 10 kg/h Vinylpyrrolidon/Vinylacetat (6:4)-Copolymer (30 Gew.-%) mit 40 Gew.-% Mannit und 30 Gew.-% Verapamil aufgeschmolzen. Parallel dazu werden über einen weiteren zweiwelligen Extruder (ZSK 30) 30 kg/h Hydroxypropylcellulose, die als Wirkstoff 30 Gew.-% Verapamil enthält, aufbereitet. Die beiden Stränge werden über eine kreisringspaltförmige Koextrusionsdüse unter den in Beispiel 1 genannten Bedingungen extrudiert. Die Ausformung zu Tabletten erfolgt mit der in Figur 2 gezeigten Vorrichtung nach der in Beispiel 1 angegebenen Methode. Die Tabletten bestehen aus einem wirkstoffhaltigen Hydroxypropylcellulosekern und einer Vinylpyrrolidon/-Vinylacetat-Copolymer-Mannithülle.

### Beispiel 9

Nach dem in Beispiel 2 angegebenen Verfahren werden Tabletten hergestellt, die einen Hydroxypropylcellulose-Kern (niedrig substituierte Hydroxypropylcellulose, Typ LH 31) mit Vitamin A und E und eine Hydroxypropylcellulose-Hülle (Klucel F) mit Vitamin C besitzen.

## Patentansprüche

1. Verfahren zur Herstellung mehrschichtiger, fester Arzneiformen zur oralen oder rektalen Verabreichung,
**dadurch gekennzeichnet,**
**daß** man mindestens zwei Massen, die jeweils ein thermoplastisches, pharmakologisch akzeptables und in physiologischer Umgebung lösliches oder quellbares polymeres Bindemittel umfassen und von denen wenigstens eine einen pharmazeutischen Wirkstoff enthält, koextrudiert und das koextrudierte mehrschichtige Material zu der gewünschten Arzneiform formt.

2. Verfahren nach Anspruch 1, **dadurch gekennzeichnet, daß** das polymere Bindemittel für eine wirkstofffreie Schicht ausgewählt ist unter Hydroxyalkylcellulosen, Alkylcellulosen, Hydroxyalkyl-Alkylcellulosen, Cellulosephthalaten, Polyacrylaten, Galactomannanen und Polylactiden.

3. Verfahren nach Anspruch 1 oder 2, **dadurch gekennzeichnet, daß** das polymere Bindemittel für eine wirkstoffhaltige Schicht ausgewählt ist unter Polyvinylpyrrolidonen, Copolymerisaten von N-Vinylpyrrolidon und Vinylestern und Hydroxyalkylacrylaten.

4. Verfahren nach einem der vorhergehenden Ansprüche, **dadurch gekennzeichnet, daß** man einen Wirkstoff verwendet, der ausgewählt ist unter Ibuprofen, Ketoprofen, Flurbiprofen, Acetylsalicylsäure, Verapamil, Paracetamol, Nifedipin, Coffein, Captopril und Vitaminen oder Mischungen von zwei oder mehreren dieser Wirkstoffe.

5. Verfahren nach einem der vorhergehenden Ansprüche, **dadurch gekennzeichnet, daß** man zur Herstellung geschlossener Arzneiformen die Koextrusion mit einer konzentrischen kreisringspaltförmigen Koextrusionsdüse und die Formung in einem Formkalander oder durch Heiß- oder Kaltabschlag durchführt.

6. Verfahren nach einem der Ansprüche 1 bis 4, **dadurch gekennzeichnet, daß** man zur Herstellung offener Arzneiformen, insbesondere offener Mehrschichttabletten, die Koextrusion unter Verwendung einer Breitschlitzdüse und die Formung durch Ausstanzen durchführt.

7. Verfahren nach Anspruch 5 oder 6, **dadurch gekennzeichnet, daß** man als polymeres Bindemittel für die Außenschicht(en) Hydroxypropyl-Cellulose und für die Innenschicht bzw. den Kern Polyvinylpyrrolidon verwendet.

8. Verfahren nach einem der Ansprüche 5 bis 7, **dadurch gekennzeichnet, daß** man als Wirkstoff Ibuprofen alleine oder Ibuprofen/Coffein oder die Vitamine A, C, E verwendet, wobei sich das Ibuprofen bzw. die Vitamine A und E im Kern und das Coffein bzw. das Vitamin C in der Außenschicht befinden.

9. Verfahren nach einem der vorhergehenden Ansprüche, daß man die mehrschichtigen Arzneiformen in einem anschließenden Verfahrensschritt rundiert und/oder mit einem Coating versieht.

10. Mehrschichtige feste Arzneiform zur oralen oder rektalen Verabreichung, erhältlich nach einem Verfahren gemäß einem der Ansprüche 1 bis 9.

## Claims

1. A process for producing multilayer, solid drug forms for oral or rectal administration, which comprises coextrusion of at least two compositions which in each case comprise a thermoplastic, pharmacologically acceptable polymeric binder which is soluble or swellable in a physiological environment, and at least one of which contains a pharmaceutical active ingredient, and shaping the coextruded multilayer material to the required drug form.

2. A process as claimed in claim 1, wherein the polymeric binder for a layer containing no active ingredient is selected from among hydroxyalkylcelluloses, alkylcelluloses, hydroxyalkylalkylcelluloses, cellulose phthalates, polyacrylates, galactomannans and polylactides.

3. A process as claimed in claim 1 or 2, wherein the polymeric binder for a layer containing active ingredient is selected from among polyvinylpyrrolidones, copolymers of N-vinylpyrrolidone and vinyl esters and hydroxyalkyl acrylates.

4. A process as claimed in any of the preceding claims, wherein an active ingredient which is selected from among ibuprofen, ketoprofen, flurbiprofen, acetylsalicylic acid, verapamil, paracetamol, nifedipine, caffeine, captopril and vitamins or mixtures of two or more of these active ingredients is used.

5. A process as claimed in any of the preceding claims, wherein to produce closed drug forms the coextrusion is carried out with a concentric annular coextrusion die and the shaping is carried out in a molding calender or by a hot or cold cut method.

6. A process as claimed in any of claims 1 to 4, wherein to produce open drug forms, especially open multilayer tablets, the coextrusion is carried out using a slot die and the shaping is carried out by punching.

7. A process as claimed in claim 5 or 6, wherein hydroxypropylcellulose is used as polymeric binder for the outer layer(s), and polyvinylpyrrolidone is used for the inner layer and the core, respectively.

8. A process as claimed in any of claims 5 to 7, wherein ibuprofen alone or ibuprofen/caffeine or vitamins A, C, E are used as active ingredient, with the ibuprofen or the vitamins A and E being located in the core and the caffeine or the vitamin C being located in the outer layer.

9. A process as claimed in any of the preceding claims, wherein the multilayer drug forms are, in a subsequent step, rounded and/or provided with a coating.

10. A multilayer solid drug form for oral or rectal administration obtainable by a process as claimed in any of claims 1 to 9.

## Revendications

1. Procédé pour la préparation de formes galéniques solides, multicouche pour administration orale ou rectale,
**caractérisé par le fait**
**qu'**on co-extrude au moins deux masses, qui comportent chacune un liant polymère thermoplastique, pharmacologiquement acceptable et soluble ou gonflable dans un environnement physiologique, et dont au moins l'une contient une substance active pharmaceutique, et on élabore la matière multicouche co-extrudée sous la forme galénique souhaitée.

2. Procédé selon la revendication 1, **caractérisé par le fait que** le liant polymère pour une couche exempte de substance active est choisi parmi les hydroxyalkylcelluloses, les alkylcelluloses, les hydroxyalkyl-alkylcelluloses, les phtalates de cellulose, les polyacrylates, les galactomannanes et les polylactides.

3. Procédé selon la revendication 1 ou 2, **caractérisé par le fait que** le liant polymère pour une couche contenant une substance active est choisi parmi les poly(pyrrolidones de vinyle), les copolymères de N-pyrrolidone de vinyle et d'esters de vinyle et les acrylates d'hydroxyalkyle.

4. Procédé selon l'une des revendications précédentes, **caractérisé par le fait qu'**on utilise une substance active qui est choisie parmi l'ibuprofène, le kétoprofène, le flurbiprofène, l'acide acétylsalicylique, le vérapamil, la paracétamol, la nifédipine, la caféine, le captopril et les vitamines ou des mélanges de deux ou plusieurs de ces substances actives.

5. Procédé selon l'une des revendications précédentes, **caractérisé par le fait que**, pour la préparation de formes galéniques fermées, on effectue la co-extrusion avec une filière de co-extrusion concentrique ayant la forme d'une fente en couronne circulaire et le formage dans une calandre de profilage ou par abattage à chaud ou à froid.

6. Procédé selon l'une des revendications 1 à 4, **caractérisé par le fait que**, pour la préparation de formes galéniques ouvertes, en particulier de comprimés multicouches ouverts, on effectue la co-extrusion en employant une filière plate et le formage par estampage.

7. Procédé selon la revendication 5 ou 6, **caractérisé par le fait qu'**on utilise de l'hydroxycellulose comme liant polymère pour la(les) couche(s) externe(s) et de la poly(pyrrolidone de vinyle) pour la couche interne ou le noyau.

8. Procédé selon l'une des revendications 5 à 7, **caractérisé par le fait qu'**on utilise comme substance active l'ibuprofène seul ou de l'ibuprofène/caféine ou les vitamines A, C, E, tandis que l'ibuprofène ou les vitamines A et E se trouvent dans le noyau et la caféine ou la vitamine C dans la couche externe.

9. Procédé selon l'une des revendications précédentes, **caractérisé par le fait qu'**on arrondit les formes galéniques multicouches dans une étape opératoire subséquente et/ou on les munit d'un revêtement.

10. Forme galénique solide, multicouche pour administration orale ou rectale, pouvant être obtenue par le procédé selon l'une des revendications 1 à 9.
